# EUROPÄISCHE PATENTSCHRIFT

(11) **EP 2 542 279 B1**
(45) Veröffentlichungstag und Bekanntmachung des Hinweises auf die Patenterteilung: **05.11.2014**
(21) Anmeldenummer: 10801418.4
(22) Anmeldetag: 23.12.2010
(51) Int. Cl.: A61M 5/142, F04B 43/08, F04B 43/12

(54) **SCHLAUCHPUMPE MIT PLANETENGETRIEBE**
HOSE PUMP WITH PLANETARY GEAR
POMPE PÉRISTALTIQUE À ENGRENAGE PLANÉTAIRE

(30) Priorität: 01.03.2010 DE 102010000592
(43) Veröffentlichungstag der Anmeldung: 09.01.2013
(73) Patentinhaber: Ulrich GmbH & Co. KG, 89081 Ulm (DE)
(72) Erfinder: ZUPP, Andre, 06847 Dessau (DE); SCHWERDTFEGER, Uwe, 39439 Amesdorf (DE)
(74) Vertreter: Charrier, Rapp & Liebau
(86) Internationale Anmeldenummer: PCT/EP2010/070714
(87) Internationale Veröffentlichungsnummer: WO 2011/107179

(56) Entgegenhaltungen:
- WO-A1-02/25112
- FR-A5- 2 071 238
- US-A- 3 358 609
- US-A- 3 749 531
- US-A- 4 604 038
- US-A- 5 941 696
- US-A1- 2005 129 545

## Beschreibung

Die Erfindung betrifft eine Schlauchpumpe nach dem Oberbegriff des Anspruchs 1.

Derartige Schlauchpumpen werden insbesondere im Bereich der Medizintechnik, beispielsweise als Infusionspumpe, oder in Injektions- und Dialysegeräten verwendet. Eine gattungsgemäße Schlauchpumpe ist beispielsweise aus der Patentschrift AT 367 874 bekannt. Darin ist eine Schlauchpumpe mit mehreren über ein Planetengetriebe von einem Zentralteil antreibbaren Rollen beschrieben, bei der die Rollen auf zumindest einem Schlauch, in dem das zu fördernde Medium geführt wird, unter Quetschung von dessen freiem Querschnitt abrollen. Die Rollen sind drehbar auf einem drehbar gelagerten Träger angeordnet und stehen zumindest mit einem Teil ihres Umfangs während des Anliegens an dem Schlauch mit dem Zentralteil in reibungsschlüssigem Kontakt. Die Schlauchpumpe umfasst eine mit dem Zentralteil drehfest verbundene Welle, welche von einem Motor antreibbar ist. Das Zentralteil weist zwei umlaufende Rillen auf, in denen elastisch verformbare Gummiringe gehalten sind, welche am Außenumfang der Rollen anliegen und diese bei rotierendem Zentralteil nach Art eines Planetengetriebes reibungsschlüssig antreiben. Der Träger, auf dem die Rollen drehbar gelagert sind, ist durch zwei in axialem Abstand zueinander am Zentralteil drehbar gelagerte Scheiben gebildet. Wird die Welle von dem Motor angetrieben, so werden die Rollen über die Gummiringe von dem Zentralteil ebenfalls in Drehung versetzt. Die Schläuche liegen an einem Gegendruckkörper an und werden durch die Rollen gegen den Gegendruckkörper gequetscht, wodurch bei laufender Pumpe das zwischen zwei Quetschstellen eingeschlossene Volumen in dem Schlauch während des Abrollens der Rollen an den Schläuchen weitergefördert wird.

Die Einführung eines Schlauchs in diese bekannte Schlauchpumpe erweist sich als schwierig und zeitaufwendig, denn der Schlauch bzw. die Schläuche müssen mühsam zwischen dem Außenumfang der Rollen und dem Gegendruckkörper eingefädelt werden.

Aus der FR 2 071 238, die als nächstliegender Stand der Technik angesehen wird, ist eine Schlauchpumpe mit einem Gehäuse, einem Antrieb und mehreren Quetschrollen bekannt, welche von dem Antrieb über ein Getriebe mit einem Sonnenrad und einem Drehfest mit der jeweiligen Quetschrolle verbundenen ersten Planetenrad antreibbar sind, wobei die sich drehenden Quetschrollen bei laufender Pumpe einen in die Pumpe eingelegten Schlauch unter Quetschung des Schlauchs gegen ein Gegenlager drücken und dadurch ein sich in dem Schlauch befindliches Medium in Förderrichtung fördern. Jeder Quetschrolle ist neben dem ersten Planetenrad ein zweites Planetenrad zugeordnet.

Die US 3 749 531 offenbart eine Schlauchpumpe mit einem Träger, auf dem Quetschrollen drehbar gelagert sind.

Aus der DE 71 13 125-U ist eine Schlauchquetschpumpe zur stoßfreien Förderung von flüssigen, fließfähigen und/oder gasförmigen Medien bekannt, welche aus einem Rollenkörper mit mehreren, kreisförmig angeordneten, eine kreisförmige Abrollumlaufbahn beschreibenden und in Gegenrichtung zur Abrollumlaufbahn angetriebenen Andruckrollen zum Zusammendrücken und wieder freigeben eines oder mehrerer Förderschläuche,

Diese Aufgaben werden mit einer Schlauchpumpe mit den Merkmalen des Anspruchs 1 und mit einer Schlauchpumpe mit den Merkmalen des Anspruchs 12 gelöst. Bevorzugte Ausführungsformen der erfindungsgemäßen Schlauchpumpe sind den Unteransprüchen zu entnehmen.

Bei der erfindungsgemäßen Schlauchpumpe wird eine einfachere und schnellere Einfädelung eines Schlauches zwischen die Quetschrollen und dem Gegenlager bei laufender Pumpe ermöglicht, indem jeder Quetschrolle ein erstes Planetenrad und ein zweiten Planetenrad zugeordnet ist, wobei das erste Planetenrad jeder Quetschrolle mit einem von dem Antrieb drehend antreibbaren Sonnenrad gekoppelt ist, um die Quetschrollen bei laufender Pumpe in Drehung zu versetzen und wobei jeweils das zweite Planetenrad jeder Quetschrolle mit dem als Hohlrad wirkenden Innenumfang des Gehäuses gekoppelt ist. Bei laufender Pumpe wird durch diese Anordnung sowohl der Träger als auch die darauf drehbar gelagerten Quetschrollen in Drehung versetzt. Durch die Drehung des Trägers gegenüber dem Gehäuse kann ein Schlauch einfach in die Schlauchpumpe zwischen den Quetschrollen und dem Gegenlager eingefädelt werden, indem der Schlauch oder ein Schlauchende eingangsseitig der Schlauchpumpe eingefügt wird. Durch die Drehung des Trägers wird die dem eingelegten Schlauch am nächsten liegende Quetschrolle in Richtung des eingelegten Schlauchs bewegt, wodurch der Schlauch zwischen dem Außenumfang dieser Quetschrolle und dem Gegenlager eingequetscht und durch Haftreibung an der Schlauchoberfläche weiter in die Schlauchpumpe eingezogen wird. Durch die weitere Drehung des Trägers gegenüber dem Gehäuse erfolgt dies in entsprechender Weise auch bei den übrigen Quetschrollen, bis der Schlauch vollständig in die Schlauchpumpe eingezogen worden ist.

Die Erfindung geht dabei davon aus, dass eine selbsttätige Einfädelung des Schlauchs in die Schlauchpumpe dadurch ermöglicht wird, dass sich auch bei noch nicht eingelegtem Schlauch bei laufender Pumpe sowohl der Träger als auch die darauf gelagerten Quetschrollen drehen. Bei der eingangs beschriebenen Schlauchpumpe des Stands der Technik dreht sich der Träger nur dann, wenn bereits ein Schlauch zwischen den Quetschrollen und dem Gegenlager eingeführt ist. Um auch bei noch nicht eingelegtem Schlauch eine Drehung sowohl des Trägers als auch der auf dem Träger gelagerten Quetschrollen zu ermöglichen, ist nach der Erfindung jede Quetschrolle mit einem ersten Planetenrad und einem zweiten Planetenrad ausgestattet, wobei das erste Planetenrad, welches mit dem Sonnenrad unmittelbar gekoppelt ist, die Quetschrolle in Rotation versetzt und das zweite Planetenrad das Drehmoment des Sonnenrads über den als Hohlrad wirkenden Innenumfang des feststehenden Gehäuses auf den Träger überträgt und dadurch den Träger bei laufender Pumpe auch ohne eingelegten Schlauch in Drehung versetzen kann.

In einem bevorzugten Ausführungsbeispiel ist der Träger als Trägerscheibe ausgebildet und über ein Lager drehbar an einer Antriebswelle des Antriebs gelagert. Die Quetschrollen sind zweckmäßig jeweils auf einer drehbar in der Trägerscheibe gelagerten Quetschrollenwelle angeordnet. Die beiden Planetenräder jeder Quetschrolle sind dabei in axialem Abstand zueinander drehfest an der jeweiligen Quetschrollenwelle befestigt. Das erste Planetenrad jeder Quetschrolle steht bevorzugt über eine Verzahnung mit dem Sonnenrad in Kontakt, so dass das Drehmoment des Sonnenrads jeweils über das erste Planetenrad auf die diesem zugeordnete Quetschrolle übertragen wird, um die Quetschrollen bei laufender Pumpe in Rotation zu versetzen. Durch die Verzahnung können hohe Drehmomente übertragen werden. Das zweite Planetenrad jeder Quetschrolle steht mit dem Innenumfang des Gehäuses in Kontakt und rollt bei laufender Pumpe am Innenumfang des Gehäuses ab, wodurch das Drehmoment des Sonnenrads über das zweite Planetenrad jeder Quetschrolle auf die Trägerscheibe übertragen wird. Hierfür sind die Quetschrollen jeweils in der Peripherie der Trägerscheibe angeordnet und dort drehbar gelagert.

Ist noch kein Schlauch in der Pumpe eingelegt, treibt der Antrieb das Sonnerad an und die Planetenräder übertragen das Drehmoment des Sonnenrads auf die Quetschrollen, welche sich in der durch die Reibrollen definierten Umfangsgeschwindigkeit drehen. Gleichzeitig wird dieTrägerscheibe durch das Abrollen des Reibrads bzw. der Reibräder an dem als Hohlrad wirkenden Innenumfang des feststehenden Gehäuses in Drehung versetzt.

Ist ein Schlauch in der Pumpe eingelegt, treibt der Motor das Sonnerad an und dieses überträgt das Drehmoment über die Planetenräder auf die Quetschrollen. Die Reibräder haben dabei keine Wirkung mehr, denn sie rutschen am Innenumfang des Gehäuses durch. Die Drehmomentübertragung erfolgt direkt vom Sonnenrad auf die Quetschrollen, welche an der Oberfläche des eingelegten Schlauchs abrollen und diesen gegen das Gegenlager quetschen. Dadurch wird wiederum die Trägerscheibe in Rotattion versetzt. Die Umfangsgeschwindigkeit der Quetschrollen stellt sich dabei auf ein ideales Maß ein. Insbesondere wird dadurch einerseits verhindert, dass durch zu schnelle Rollenbewegung der Schlauch nicht mit der Drehbewegung der Trägerscheibe vor der Quetschrolle hergeschoben wird. Bei (zu) langsamer Rollenbewegung wird andererseits der Schlauch nicht entgegen der Drehbewegungen der Tägerscheibe "zurück" gedrückt. In beiden Fällen wird eine übermäßige Beanspruchung des Schlauchs sowie Reibungsverluste vermieden, weshalb bei der erfindungsgemäßen Pumpe bei gleicher Pumpleistung der Motor mit geringerer Leistung betrieben werden kann.

Das zweite Planetenrad jeder Quetschrolle ist bevorzugt als zahnfreies Reibrad ausgebildet, welches an dem ebenfalls zahnfrei bzw. glatt ausgebildeten Innenumfang des Gehäuses abrollt. Hierfür weist jedes zweite Planetenrad zweckmäßig an seinem Außenumfang einen Ring aus einem elastomeren Material auf (bspw. einen O-Ring aus Gummi), über den jedes zweite Planetenrad mit dem Innenumfang des Gehäuses in reibschlüssigem Kontakt steht. Durch die relativ geringe Reibkraft zwischen dem Reibrad und dem als Hohlrad wirkenden Innenumfang des Gehäuses stellt sich selbsttätig eine optimale Umfangsgeschwindigkeit der Trägerscheibe und der Quetschrollen ein.

Um ein selbsttätiges Einfädeln eines Schlauchs in die Schlauchpumpe zu ermöglichen, ist in einem bevorzugten Ausführungsbeispiel eine Einfädeleinrichtung vorgesehen, welche einen Schlauch bei laufender Pumpe automatisch in die Schlauchpumpe zwischen dem Außenumfang der Quetschrollen und dem Gegenlager einfädelt. Die Einfädeleinrichtung umfasst bevorzugt eine von einem Spindelantrieb drehend antreibbare Schneckenspindel. Der Spindelantrieb ist zweckmäßig so mit dem Antrieb der Pumpe gekoppelt, dass der Spindelantrieb die Schneckenspindel in Drehung versetzt, sobald der Antrieb der Pumpe die Trägerscheibe in Rotation versetzt. Zur Einfädelung eines Schlauchs in die Schlauchpumpe ist es lediglich noch erforderlich, einen Schlauch oder ein Schlauchende in die Schneckenspindel einzulegen und die Pumpe in Gang zu setzen. Beim Starten der Pumpe wird auch bei noch nicht eingeführtem Schlauch die Trägerscheibe und die darauf gelagerten Quetschrollen in Drehung versetzt. Gleichzeitig fädelt die Einfädeleinrichtung den darin eingefügten Schlauch in Richtung einer der Schneckenspindel benachbarten Quetschrolle in die Schlauchpumpe ein.

Um die selbsttätige Einfädelung des Schlauchs in die Schlauchpumpe durch die Einfädeleinrichtung zu unterstützten ist neben den Quetschrollen zusätzlich noch wenigstens eine Führungsrolle vorgesehen. Bevorzugt sind mehrere Führungsrollen vorgesehen, welche jeweils zwischen benachbarten Quetschrollen auf der Trägerscheibe drehbar angeordnet sind. Die Führungsrollen weisen am Außenumfang eine umlaufende Nut auf, in welche der Schlauch eingreifen kann. Wenn der Schlauch von der Einfadeleinrichtung nach unten in Richtung der Trägerscheibe geführt wird, greift er in die Nut am Außenumfang derjenigen Führungsrolle ein, die gerade benachbart zur Einfädeteinrichtung liegt. Durch die Drehung der Trägerscheibe bewegt sich die darauf angeordnete Führungsrolle in Förderrichtung der Pumpe weiter und zieht dabei den Schlauch zum einen nach unten in Richtung der Trägerscheibe und drückt ihn zum anderen in radialer Richtung nach außen gegen das Gegenlager. Bei weiterer Drehung der Trägerscheibe zieht die Führungsrolle den Schlauch aufgrund der Haftreibung an der Schlauchoberfläche und des Kraftschlusses in der Nut an ihrem Außenumfang weiter in die Schlauchpumpe entlang des Innenumfangs des kreissegmentförmig ausgebildeten Gegenlagers ein, bis die Trägerscheibe mit der darauf angeordneten Führungsrolle (nahezu) eine volle Umdrehung ausgeführt hat und der Schlauch durch die weitere Drehung der Trägerscheibe vollständig in die Schlauchpumpe eingezogen ist.. Durch die Drehung der Trägerscheibe wird der Schlauch von der auf der Trägerscheibe der Führungsrolle folgenden Quetschrolle schließlich gegen das Gegenlager gequetscht und so zwischen der Quetschrolle und dem Gegenlager geklemmt. Dies erfolgt bei weiterer Drehung der Trägerscheibe auch bei den übrigen Führungs- und Quetschrollen in entsprechender Weise, bis der Schlauch vollständig in die Schlauchpumpe eingezogen ist.

Weitere Vorteile und Besonderheiten der Erfindung ergeben sich aus dem nachfolgend unter Bezugnahme auf die begleitenden Zeichnungen beschriebenen Ausführungsbeispiel. Die Zeichnungen zeigen:
- **Figur 1:**: Draufsicht auf eine Injektionsvorrichtung, in der eine erfindungsgemäße Schlauchpumpe verwendet wird;
- **Figur 2:**: Perspektivische Darstellung einer erfindungsgemäßen Schlauchpumpe;
- **Figur 3:**: Explosionsdarstellung der Schlauchpumpe von Figur 2;
- **Figur 4a:**: Schnittdarstellung der Schlauchpumpe von Figur 2 entlang der Ebene A-A;
- **Figur 4b:**: Detailansicht einer Schnittdarstellung der Schlauchpumpe von Figur 2 im Bereich des Gegenlagers und einer dem Gegenlager gegenüberliegenden Führungsrolle;

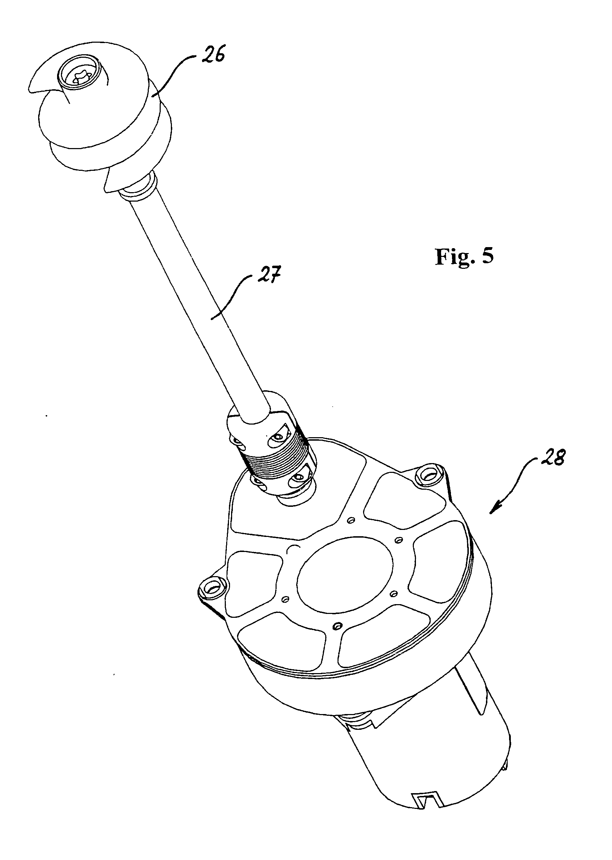

- **Fig. 5:**: Perspektivische Darstellung der Einfädeleinrichtung der Schlauchpumpe von Fig. 2.

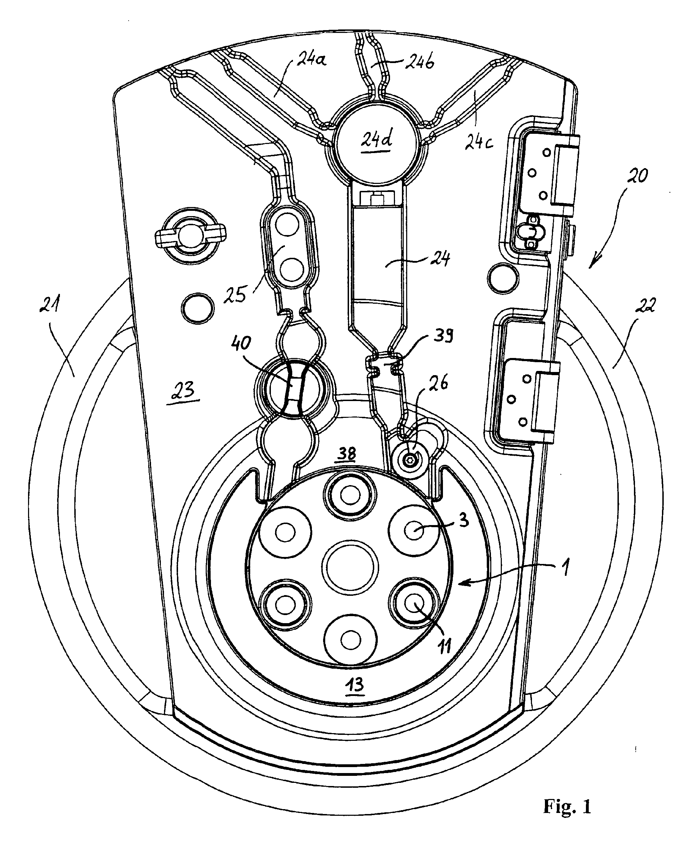

In Figur 1 ist der Injektionskopf einer Injektionsvorrichtung zur Injektion von zwei verschiedenen oder gleichen Kontrastmitteln und einer NaCl-Spüllösung in die Blutbahn eines Patienten gezeigt, in der eine erfindungsgemäße Schlauchpumpe 1 verwendet wird. Solche Injektionsvorrichtungen werden bspw. zur Injektion von Kontrastmitteln bei der Durchführung von bildgebenden Verfahren wie Computertomographie, Ultraschalluntersuchungen und Magnetresonanztomographie (MRT) verwendet. Die Injektionsvorrichtung umfasst den in Figur 1 gezeigten Injektionskopf 20, in dem die Schlauchpumpe 1 angeordnet ist. Der Injektionskopf 20 umfasst ein Kunststoffgehäuse mit zwei kreisringförmigen Handgriffen 21, 22. Zwischen den Handgriffen 21 und 22 ist ein Panel 23 angeordnet, welches mit einem hier zeichnerisch nicht dargestellten Deckel verschließbar ist. Das Panel 23 weist in seinem unteren Bereich eine Ausnehmung zur Aufnahme der Schlauchpumpe 1 auf. Darüber befinden sich kanalförmige Ausnehmungen 24, 25, in welche eine verzweigte Schlauchanordnung (welche hier zeichnerisch nicht dargestellt ist) eingefügt werden kann. Bei der Schlauchanordnung handelt es sich insbesondere um eine Schlauchanordnung, wie sie in der EP 2 011 541 A2 im Detail beschrieben ist. Diese Schlauchanordnung umfasst insgesamt drei Zufuhrschläuche, nämlich einen ersten Zufuhrschlauch für ein erstes Kontrastmittel, einen zweiten Zufuhrschlauch für ein zweites Kontrastmittel und einen dritten Zufuhrschlauch für eine Spüllösung (insbesondere NaCl). Die drei Zufuhrschläuche werden an hier ebenfalls zeichnerisch nicht dargestellte Vorratsflaschen für die Kontrastmittel und die Spüllösung angeschlossen und in die im oberen Bereich des Panels 23 angeordneten Verzweigungen 24a, 24b und 24c der Ausnehmung 24 eingefügt. Die drei von den Vorratsbehältern kommenden Zufuhrschläuche werden über ein Verzweigungsstück, welches in der kreisförmigen Ausnehmung 24d des Panels 23 eingefügt wird, in einem Schlauchstück zusammengeführt, welches zur Schlauchpumpe 1 geführt wird.

Zur Einführung des Schlauchs in die Schlauchpumpe 1 ist eine Einfädeleinrichtung vorgesehen. Die Funktionsweise und die Ausgestaltung dieser Einfädeleinrichtung wird im Folgenden noch erläutert. Der Schlauch wird schließlich durch die Schlauchpumpe 1 geführt und in die Ausnehmung 25 im oberen, linken Teil des Panels 23 eingelegt. Das Schlauchende wird an einen Patientenschlauch angeschlossen, über den die in dem Schlauch geführten Medien schließlich in die Blutbahn des Patienten injiziert werden können. Zur Fixierung des Schlauchs auf dem Panel 23 ist eine Fixiereinrichtung vorgesehen, welche eine Fixierung des Schlauchs an einer ersten, eingangsseitigen Stelle 39 und wenigstens einer zweiten, ausgangseitigen Stelle 40 der Schlauchpumpe ermöglicht. An den Fixierstellen 39 und 40 sind zweckmäßig Ultraschallsensoren zur Erfassung von Luftblasen in dem Schlauch angeordnet. Weitere Fixierstellen des Schlauchs auf dem Panel 23 sind möglich und bspw. in der EP 2011541 A1 beschrieben.

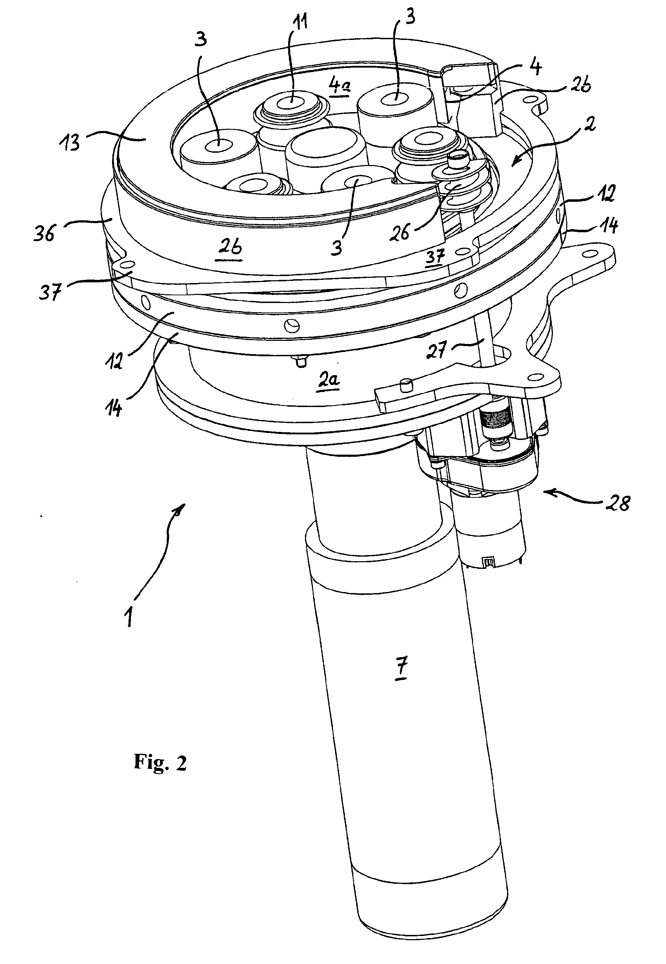

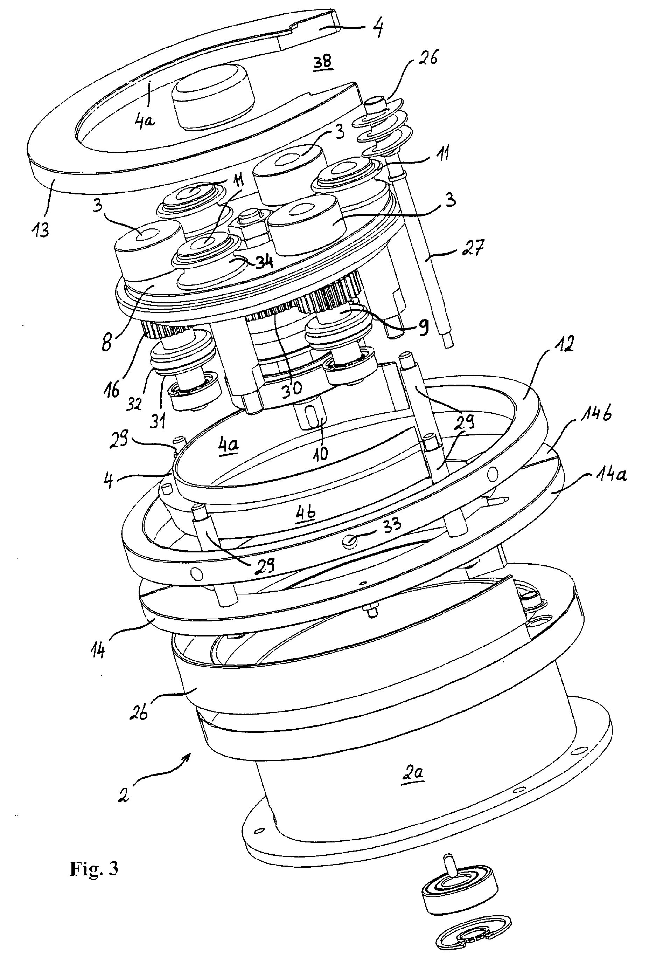

In den Figuren 2 und 3 ist die Schlauchpumpe 1 im Detail in einer perspektivischen Ansicht gezeigt, wobei Figur 3 eine Explosionsdarstellung ist. Die Schlauchpumpe 1 umfasst eine untere Pumpeneinheit mit einem Antriebsmotor 7, sowie eine obere Pumpeneinheit mit einem Gehäuse 2. Das Gehäuse 2 ist in einen unteren Gehäuseteil 2a und einen oberen Gehäuseteil 2b unterteilt. Der untere Gehäuseteil 2a kann mit dem oberen Gehäuseteil 2b einstückig oder auch zweiteilig ausgebildet sein.

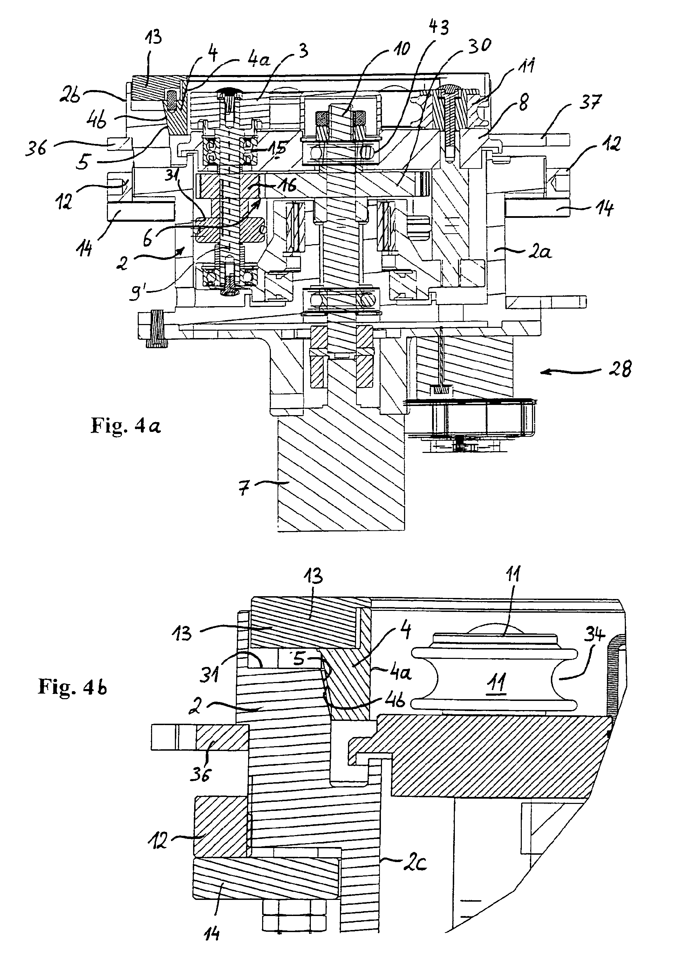

Die untere Pumpeneinheit umfasst den Antriebsmotor 7 mit einer Antriebswelle 10, welche über ein Getriebe mit der oberen Pumpeneinheit gekoppelt ist. Der Aufbau der oberen Pumpeneinheit ist aus der Schnittdarstellung der Figur 4 zu entnehmen. Im Innern des Gehäuses 2 ist ein an die Antriebswelle 10 des Antriebsmotors 7 gekoppeltes Getriebe 6 angeordnet. Das Getriebe umfasst ein Sonnenrad 30, welches drehfest mit der Antriebswelle 10 des Antriebsmotors 7 verbunden ist. Das obere Ende der Antriebswelle 10 ist über ein Lager 43 in einer Trägerscheibe 8 drehbar gelagert. Auf der Trägerscheibe 8 sind mehrere Quetschelemente 3 angeordnet. Bei den Quetschelementen 3 handelt es sich bei dem hier zeichnerisch dargestellten Ausführungsbeispiel um angetriebene Quetschrollen 3, wobei hier drei solcher Quetschrollen 3 gleichförmig am Außenumfang der kreisrunden Trägerscheibe 8 angeordnet sind. Die Quetschrollen 3 sind drehbar auf der Trägerscheibe 8 gelagert. Hierfür ist jede der drei Quetschrollen 3 auf einer Welle 9 mit einer Achse 9' aufgesetzt und jede Welle 9 ist über ein Lager 15 in einer Bohrung der Trägerscheibe 8 gelagert. Die Wellen 9 und damit die Achsen 9' der Quetschrollen 3 verlaufen parallel zur Antriebswelle 10 des Antriebsmotors 7. Der Antriebsmotor 7 versetzt die Trägerscheibe 8 und die Quetschrollen 3 bei laufender Pumpe über das Getriebe 6 in Drehung. Das Getriebe 6 umfasst neben dem Sonnenrad 30 Planetenräder 16, wobei jeder Quetschrolle 3 ein solches Planetenrad 16 zugeordnet und drehfest an der Welle 9 befestigt ist. Jedes der Planetenräder 16 ist über eine Zahnung an das Sonnenrad 30 des Planetengetriebes gekoppelt. An jeder Welle 9 ist neben dem Planetenrad 16 ein Reibrad 31 angeordnet, wobei das Reibrad 31 drehfest und im Abstand zum Planetenrad 16 an der Welle 9 befestigt ist. Am Außenumfang jedes Reibrads 31 ist eine umlaufende Nut 34 angeordnet, in welche ein Gummiring 32 (O-Ring) eingefügt ist. Über diesen Gummiring 32 steht das Reibrad 31 mit dem Innenumfang 2c des Pumpengehäuses 2 in Kontakt. Der Innenumfang 2c des Gehäuses 2 wirkt dadurch als Hohlrad eines Planetengetriebes. Wird die Antriebswelle 10 durch den Antriebsmotor 7 in Rotation versetzt, so wird diese Rotationsbewegung über die Kopplung des Planetenrads 16 am Sonnenrad 30 auf die Welle 9 übertragen, wodurch sich die Welle 9 und die drehfest mit dieser verbundene Quetschrolle 3 in Rotation versetzt. Gleichzeitig rollt das Reibrad 31 am Innenumfang 2c des Pumpengehäuses 2 ab, wodurch sich die Trägerscheibe 8 ebenfalls gegenüber dem Pumpengehäuse 2 in Drehung versetzt. Durch die Reibräder 31 kann die Trägerscheibe 8 von dem Antriebsmotor 7 auch dann in Rotation versetzt werden, wenn sich noch kein Schlauch in der Schlauchpumpe befindet.

Auf der Trägerscheibe 8 sind zusätzlich zu den Quetschrollen 3 noch Führungsrollen 11 gelagert. Die Führungsrollen 11 dienen zur Führung des Schlauchs zwischen benachbarten Quetschrollen 3 und sind nicht angetrieben. Am Außenumfang weisen die Führungsrollen 11 eine im Querschnitt halbkreisförmige Nut 34 auf, in welcher der Schlauch geführt wird. Die Anordnung der Führungsrollen 11 und der Quetschrollen 3 auf der Trägerscheibe 8 ist insbesondere der Explosionsdarstellung der Figur 3 zu entnehmen.

Zur Einführung des Schlauchs in die Schlauchpumpe ist eine Einfädeleinrichtung vorgesehen, welche den Schlauch selbsttätig zwischen den Quetschrollen 3 und dem Gegenlager 4 einfädelt. Die Einfädeleinrichtung umfasst eine außerhalb der Trägerscheibe 8 angeordnete Schneckenspindel 26. Die Schneckenspindel 26 ist auf einer Welle 27 angeordnet, wobei die Welle 27 parallel zur Achse 9' der Quetschrollen 3 verläuft. Die Welle 27 ist drehbar in einem Gehäuseteil 2 der Schlauchpumpe gelagert und an einen Spindelantrieb 28 gekoppelt, mit dem die Welle 27 und die Schneckenspindel 26 in Drehung versetzt werden kann, um einen in die Schneckenspindel eingelegten Schlauch in die Schlauchpumpe einzufädeln. Die oberen Schneckengänge der Schneckenspindel 26 ragen in Längsrichtung der Schlauchpumpe (also parallel zur Achse der Wellen 10 bzw. 27) über die Oberseite der Quetschrollen 3 und der Führungsrollen 11 hinaus.

Am oberen Ende der oberen Pumpeneinheit ist ein Gegenlager 4 angeordnet. Das Gegenlager 4 ist kreissegmentförmig mit einer Aussparung 38 ausgebildet und erstreckt sich zweckmäßig über einen Winkelbereich von 200° bis 300°. Die Schneckenspindel 26 ist im Bereich der Aussparung 38 des Gegenlagers 4 angeordnet. Das Gegenlager 4 verfügt über eine Wirkfläche 4a, welche dem Außenumfang der Quetschrollen 3 in einem Abstand d gegenüberliegt. In dem Spalt zwischen der Wirkfläche 4 und dem Außenumfang jeder Quetschrolle 3 wird der Schlauch eingefädelt.

Zum Einführen des Schlauchs in die Schlauchpumpe 1 wird das einzuführende Schlauchstück zunächst über die Fixiereinrichtung an den beiden Fixierstellen 39 und 40 auf dem Panel 23 fixiert. Das Schlauchstück zwischen den Fixiereinrichtungen 39 und 40 weist (aufgrund des Eigendralls des Schlauchstücks) dann die Form einer Schlaufe auf. Anschließend wird das Schlauchstück in die Schneckenspindel 26 eingelegt. Anschließend wird die Pumpe in Gang gesetzt, wodurch der Antriebsmotor 7 die Trägerscheibe 8 in Rotation versetzt. Gleichzeitig setzt der Spindelantrieb 28 die Schneckenspindel 26 in Drehung. Hierfür ist der Spindelantrieb 28 mit der Steuerung des Antriebsmotors 7 gekoppelt. Durch die Drehung der Schneckenspindel 26 wird der Schlauch von der Schneckenspindel 26 nach unten in Richtung der Trägerscheibe 8 geführt. Durch die Drehung der Trägerscheibe wird eine der Führungsrollen 11 auf den Schlauch zubewegt und der Schlauch greift in die Nut 34 am Außenumfang der Führungsrolle 11 ein. Durch die weitere Drehung der Trägerscheibe 8 bewegt sich die darauf angeordnete Führungsrolle 11 in Förderrichtung der Pumpe weiter und zieht dabei den Schlauch durch Kraftschluss in der Nut 34 zum einen nach unten in Richtung der Trägerscheibe 8 und drückt ihn zum anderen in radialer Richtung nach außen gegen das Gegenlager 4. Bei weiterer Drehung der Trägerscheibe 8 zieht die Führungsrolle 11 den Schlauch aufgrund der Haftreibung an der Schlauchoberfläche und des Kraftschlusses in der Nut 34 an ihrem Außenumfang weiter in die Schlauchpumpe entlang des Innenumfangs des kreissegmentförmig ausgebildeten Gegenlagers 4 ein, bis die Trägerscheibe mit der darauf angeordneten Führungsrolle 11 (nahezu) eine volle Umdrehung ausgeführt hat und der Schlauch durch die weitere Drehung der Trägerscheibe vollständig in die Schlauchpumpe eingezogen worden ist. Durch die Drehung der Trägerscheibe wird der Schlauch schließlich von der auf der Trägerscheibe 8 der Führungsrolle 11 folgenden Quetschrolle 3 schließlich gegen das Gegenlager 4 gequetscht. Auf diese Weise wird der Schlauch selbsttätig zwischen dem Außenumfang der Quetschrollen 3 und dem Gegenlager 4 eingeführt und bei weiterer Drehung der Trägerscheibe 8 zur Förderung der darin geführten Flüssigkeit gequetscht.

Ist der Schlauch vollständig in der Schlauchpumpe eingeführt, drücken die Quetschrollen 3 den Schlauch bei laufender Schlauchpumpe (also bei rotierender Trägerscheibe 8 und rotierenden Quetschrollen 3) unter Quetschung des Schlauchdurchmessers gegen die Wirkfläche 4a des Gegenlagers 4, um dadurch das in dem Schlauch geführte Medium in Förderrichtung (d.h. in Drehrichtung der Trägerscheibe 8) weiterzufördern.

Nach Beendigung des Pumpvorgangs kann die Einfädeleinrichtung bei einem erforderlichen Schlauchwechsel auch zum Ausfadeln des verbrauchten Schlauchs verwendet werden. Hierzu wird der Spindelantrieb 28 bei laufender Schlauchpumpe in umgekehrter Drehrichtung betrieben. Dadurch zieht die Schneckenspindel 26 das in die Schlauchpumpe eingelegte Schlauchstück nach oben, so dass der Eingriff des Schlauchs in der Nut 34 der Führungsrollen 11 gelöst wird. Nach einer vollen Umdrehung der Trägerscheibe ist der Schlauch vollständig aus der Schlauchpumpe heraus gezogen und kann nach Lösen der Fixierungen an den Fixierstellen 39 und 40 entnommen und durch einen neuen Schlauch ersetzt werden. Zur Einleitung der Ausfädelung eines verbrauchten Schlauchs ist eine Steuerroutine in der Steuerung des Spindelantriebs 28 vorgesehen, welche auf Knopfdruck vom Bediener ausgelöst werden kann.

Zur optimalen Einstellung des Abstands zwischen dem Gegenlager 4 und den Quetschrollen 3 ist das Gegenlager in einem bevorzugten Ausführungsbeispiel mit seiner Wirkfläche 4a gegenüber den Quetschrollen 3 verschiebbar am Gehäuse 2 angeordnet. Hierfür ist das Gegenlager 4 mit einem Druckring 13 verbunden. Bei dem Druckring 13 handelt es sich ebenfalls um einen kreissegmentförmigen Ring. Das Gegenelement 4 weist eine der Wirkfläche 4a gegenüberliegende Stellfläche 4b auf. Diese ist konisch bzw. kegelförmig ausgebildet. Die aus dem Gegenelement 4 und dem Druckring 13 bestehende Anordnung ist in der oberen Öffnung des Gehäuses 2 so angeordnet, dass sich die konische Stellfläche 4b des Gegenelements 4 gegen eine komplementär (also ebenfalls konisch bzw. kegelförmig) geformte Stützfläche 5 am Gehäuse 2 abstützt, wobei sich die Stützfläche 5 am Gehäuse 2 nach unten (also ins Gehäuseinnere) hin konisch erweitert (Figur 4, links oben).

Auf der Außenseite des Gehäuses 2 ist ein am Gehäuse befestigter Befestigungsring 36 (welche in Figur 3 aus Gründen der Übersichtlichkeit weg gelassen worden ist) mit Befestigungsflanschen 37 zur Befestigung des Gehäuses 2 am Panel 23 des Injektionskopfes 20 vorgesehen. Auf der Außenseite des Gehäuses 2 ist weiterhin im Übergangsbereich zwischen dem unteren Gehäuseteil 2a und dem oberen Gehäuseteil 2b ein Stellring 12 angeordnet. Bei dem Stellring 12 handelt es sich um einen Kreisring, der an seiner Kreisinnenfläche über ein Innengewinde verfügt. Auf der Außenseite des Gehäuses 2 ist ein zu diesem Innengewinde komplementäres Außengewinde vorgesehen. Der Stellring ist über diese Gewindeanordnung an das Gehäuse 2 derart gekoppelt, dass durch eine Verdrehung des Stellrings 12 gegenüber dem Gehäuse 2 der Stellring in axialer Richtung kontinuierlich zwischen einer oberen Grenzstellung und einer unteren Grenzstellung bezüglich des Gehäuses 2 verschiebbar ist. Zum Verdrehen des Stellrings 12 gegenüber dem Gehäuse 2 sind am Außenumfang des Stellrings 12 mehrere Bohrungen 33 vorgesehen, in die ein Stift eingreifen kann.

An der Unterseite des Stellrings 12 liegt ein Verschiebering 14 an. Der Verschiebering 14 ist aus zwei halbkreisförmigen Ringsegmenten 14a und 14b zusammengesetzt und über mehrere Bolzen 29 mit dem Druckring 13 verbunden (Figur 3).

Über die Anordnung, bestehend aus dem Gegenlager 4, dem Druckring 13, dem Verschiebering 14 und dem Stellring 12 kann der Abstand d zwischen den Quetschrollen 3 und der Wirkfläche 4a des Gegenlagers 4 eingestellt werden.

Um den Abstand d zwischen dem Außenumfang der Quetschrollen 3 und der Wirkfläche 4a zu maximieren, wird das Gegenlager 4 in seine erste (obere) Grenzstellung gebracht. Hiervon ausgehend, kann der Abstand d verkleinert werden, indem der Stellring 12 am Gehäuse 2 in Richtung seiner unteren Grenzstellung gedreht wird. Dadurch verschiebt sich der Stellring 12 von seiner oberen Grenzstellung nach unten. Dadurch wird auch der an der Unterseite des Stellrings 12 anliegende Verschiebering 14 gegenüber dem Gehäuse nach unten verschoben. Da der Verschiebering 14 über die Bolzen 29 mit dem Druckring 13 verbunden ist, verschiebt sich dadurch auch der Druckring 13 mit dem daran befestigten Gegenlager 4 nach unten. Dabei gleitet die Stellfläche 4b des Gegenlagers 4 auf der kegelförmigen Stützfläche 5 am Gehäuse 2 entlang. Bei dieser Bewegung zieht sich das kreissegmentförmige Gegenlager 4 leicht zusammen und verringert ihren Durchmesser, wodurch die Wirkfläche 4a in radialer Richtung auf die Quetschrollen 3 bzw. die Führungsrollen 11 zu gedrückt wird. Durch diese Bewegung verringert sich der Abstand d zwischen der Wirkfläche 4a und dem Außenumfang der Quetschrollen 3. Ist der Stellring 12 bei weiterer Drehung in seiner unteren Grenzstellung angelangt, sitzt die Unterseite des Druckrings 13 auf einem Sockel 31 des Gehäuses 2 auf. In dieser Stellung ist der Abstand d zwischen der Wirkfläche 4a und dem Außenumfang der Quetschrollen 3 bzw. der Führungsrollen 11 in Minimalstellung.

Durch diese Anordnung des Gegenlagers 4 kann das Spaltmaß (also der Abstand d) zwischen der Wirkfläche 4a und dem Außenumfang der Quetschrollen 3 auf einen für den Betrieb der Pumpe optimalen Wert eingestellt werden. Diese Einstellung erfolgt erstmalig vor der Inbetriebnahme der Schlauchpumpe. Zur Einstellung eines gewünschten Abstand d wird zweckmäßig eine Lehre verwendet, deren Dicke dem einzustellenden Spaltmaß entspricht und welche zwischen der Wirkfläche 4a und dem Außenumfang der Quetschrollen 3 eingelegt wird. Anschließend wird der Stellring 12 gegenüber dem Gehäuse verdreht, bis die Wirkfläche 4a und der Außenumfang der Quetschrollen 3 an den Außenflächen der Lehre anliegen. Das Spaltmaß kann im Bedarfsfall bei einer Wartung der Schlauchpumpe nachgestellt werden.

Die Erfindung ist nicht auf das beschriebene Ausführungsbeispiel beschränkt. In einem alternativen Ausführungsform der Erfindung kann auf das zweite Planetenrad auch verzichtet werden. Um bei dieser Ausführungsform bei laufender Pumpe auch bei noch nicht eingelegtem Schlauch sowohl den Träger als auch die darauf gelagerten Quetschrollen in Drehung zu versetzen, ist jeder Quetschrolle nur ein (erstes) Planetenrad zugeordnet, welches mit dem als Hohlrad wirkenden Innenumfang des Gehäuses gekoppelt ist, wodurch der Träger von dem Antrieb bei laufender Pumpe in Drehung versetzt wird. Das erste Planetenrad jeder Quetschrolle ist dabei zweckmäßig jeweils über eine Zahnung mit dem Sonnenrad gekoppelt. Die Kopplung mit dem Innenumfang des Gehäuses erfolgt dabei über die am Außenumfang des jeweiligen Planetenrads angeordnete Außenzahnung. Hierfür ist am Innenumfang des Gehäuses eine komplementär zur Außenzahnung des Planetenrads ausgebildete Innenzahnung vorgesehen, welche mit der Außenzahnung des jeweiligen Planetenrads kämmt. Bei diesem Ausführungsbeispiel wird über die Kopplung des Planetenrads mit dem Sonnenrad bei laufender Pumpe die jeweilige Quetschrolle in Rotation versetzt und über die die Kopplung des Planetenrads mit dem Innenumfang des fest stehenden Gehäuses wird gleichzeitig der Träger in Rotation versetzt. Die Innenzahnung am Innenumfang des Gehäuses wirkt dabei als Hohlrad eines aus dem Sonnenrad, den Planetenrädern und dem fest stehenden Hohlrad gebildeten Planetengetriebes.

Die Einfädeleinrichtung kann statt der Schneckenspindel auch einen Greifer mit einem linearen Antrieb aufweisen, wobei der Greifer den in die Einfädeleinrichtung eingelegten Schlauch bzw. das eingelegte Schlauchstück ergreift oder umgreift und der lineare Antrieb den Schlauch dann nach unten in Richtung der Trägerscheibe führt, so dass dieser von den Führungsrollen, wie oben beschrieben, mitgeführt und um das Gegenlager herum in die Schlauchpumpe zwischen den Quetschrollen und dem Gegenlager eingeführt werden kann. Der lineare Antrieb kann dabei über einen Linearmotor oder über einen Rotationsmotor mit Übersetzungsgetriebe zur Umsetzung der Drehbewegung in einen Linearantrieb bereit gestellt werden. Alternativ zu einem motorischen Antrieb kann auch ein bistabiler Magnet verwendet werden, mit dem die Einfädeleinrichtung in Bewegung gesetzt werden kann, um den Schlauch in die Pumpe einzuführen. Statt den Führungsrollen können auch Halteflügel zum Einsatz kommen, welche auf der Trägerscheibe angeordnet sind und das von der Einfädeleinrichtung in die Pumpe eingeführte Schlauchstück nach unten in Richtung der Trägerscheibe und radial nach außen in Richtung des Gegenlager drücken.

Der Einsatz der erfindungsgemäßen Schlauchpumpe ist ferner nicht auf Injektionsvorrichtungen beschränkt, sondern erstreckt sich auch auf andere Pumpeinrichtungen, wie z.B. Infusionspumpen.

## Patentansprüche

1. Schlauchpumpe (1) zur Förderung eines in einem Schlauch geführten Mediums, mit einem Gehäuse (2), das einen als Hohlrad wirkenden Innenumfang (2c) aufweist, einem Antrieb (7) und mehreren Quetschrollen (3), welche von dem Antrieb (7) über ein Getriebe (6) mit einem Sonnenrad (30) und einem drehfest mit der jeweiligen Quetschrolle (3) verbundenen ersten Planetenrad (16) antreibbar sind, wobei die sich drehenden Quetschrollen (3) bei laufender Pumpe einen in die Pumpe eingelegten Schlauch unter Quetschung des Schlauchs gegen ein Gegenlager (4) drücken und dadurch das Medium in dem Schlauch in Förderrichtung weiter fördern, wobei jeder Quetschrolle (3) neben dem ersten Planetenrad (16) wenigstens ein zweites Planetenrad (31) zugeordnet ist, welches als zahnfreies Reibrad ausgebildet ist,**dadurch gekennzeichnet, dass**
- die Quetschrollen (3) auf einem gegenüber dem Gehäuse drehbaren Träger (8) drehbar gelagert sind,
- und das zweite Planetenrad (31) jeweils mit dem als Hohlrad wirkenden Innenumfang (2c) des Gehäuses (2) gekoppelt ist um den Träger (8) von dem Antrieb (7) bei laufender Pumpe in Drehung zu versetzen,
- wobei das zweite Planetenrad (31) jeder Quetschrolle (3) an seinem Außenumfang einen Ring (32) aus einem elastomeren Material aufweist und über diesen Ring (32) mit dem glatt ausgebildeten Innenumfang (2c) des Gehäuses (2) in reibschlüssigem Kontakt steht.

2. Schlauchpumpe nach Anspruch 1, **dadurch gekennzeichnet, dass** das Sonnenrad (30) drehfest mit einer Antriebswelle (10) des Antriebs (7) verbunden ist.

3. Schlauchpumpe nach Anspruch 2, **dadurch gekennzeichnet, dass** das von der Antriebswelle (10) auf das Sonnenrad (30) übertragene Drehmoment vom Sonnenrad (30) und dem zweiten Planetenrad (31) über den als Hohlrad wirkenden Innenumfang (2c) des fest stehenden Gehäuses (2) auf den Träger (8) übertragen wird, wodurch der Träger (8) bei laufender Pumpe auch ohne einen eingelegten Schlauch in Drehung versetzt wird.

4. Schlauchpumpe nach einem der voranstehenden Ansprüche, **dadurch gekennzeichnet, dass** der Träger (8) als Trägerscheibe (8) ausgebildet ist, welche über ein Lager (33) drehbar an einer Antriebswelle (10) des Antriebs (7) gelagert ist.

5. Schlauchpumpe nach Anspruch 4, **dadurch gekennzeichnet, dass** die Quetschrollen (3) jeweils auf einer drehbar in der Trägerscheibe (8) gelagerten Quetschrollenwelle (9) angeordnet sind, wobei die Achse (9') jeder Quetschrollenwelle (9) parallel zu der Antriebswelle (10) des Antriebs (7) verläuft.

6. Schlauchpumpe nach einem der voranstehenden Ansprüche, **dadurch gekennzeichnet, dass** jeweils das erste Planetenrad (16) jeder Quetschrolle (3) mit dem Sonnenrad (30) über eine Verzahnung in Kontakt steht.

7. Schlauchpumpe nach einem der voranstehenden Ansprüche, **dadurch gekennzeichnet, dass** das zweite Planetenrad (31) jeder Quetschrolle (3) mit dem Innenumfang (2c) des Gehäuses (2) in Kontakt steht und bei laufender Pumpe am Innenumfang (2c) abrollt.

8. Schlauchpumpe nach einem der Ansprüche 4 bis 7, **dadurch gekennzeichnet, dass** auf der Trägerscheibe (8) zwischen zwei benachbarten Quetschrollen (3) jeweils eine Führungsrolle (11) angeordnet ist.

9. Schlauchpumpe nach einem der Ansprüche 5 bis 8, **dadurch gekennzeichnet, dass** jedes der Planetenräder (16, 31) drehfest an der Quetschrollenwelle (9) der jeweils zugeordneten Quetschrolle (3) angeordnet ist.

10. Schlauchpumpe nach einem der voranstehenden Ansprüche, **dadurch gekennzeichnet, dass** die Schlauchpumpe über eine Einfädeleinrichtung zum Einführen des Schlauchs zwischen den Quetschelementen (3) und dem Gegenlager (4) verfügt.

11. Schlauchpumpe nach Anspruch 10, **dadurch gekennzeichnet, dass** die Einfädeleinrichtung eine Schneckenspindel (26) umfasst, welche von einem Spindelantrieb (28) drehend antreibbar ist.

12. Schlauchpumpe nach Anspruch 11" **dadurch gekennzeichnet, dass** der Spindelantrieb (28) so mit dem Antrieb (7) gekoppelt ist, dass der Spindelantrieb (28) die Schneckenspindel (26) in Drehung versetzt, sobald der Antrieb (7) die Trägerscheibe (8) in Rotation versetzt.

13. Schlauchpumpe nach einem der Ansprüche 10 bis 12, **dadurch gekennzeichnet, dass** die Einfädeleinrichtung einen darin eingelegten Schlauch bei laufender Schlauchpumpe selbsttätig zwischen den Quetschelementen (3) und dem Gegenlager (4) einführt.

14. Schlauchpumpe nach einem der voranstehenden Ansprüche, **dadurch gekennzeichnet, dass** eine Fixiereinrichtung zur Fixierung eines in die Schlauchpumpe einzuführenden Schlauchs vorgesehen ist, wobei die Fixiereinrichtung eine Fixierung des Schlauchs an einer ersten, eingangsseitigen Stelle (39) und einer zweiten, ausgangseitigen Stelle (40) der Schlauchpumpe ermöglicht.

## Claims

1. Hose pump (1) for conveying a medium guided in a hose, with a housing (2), which has an inner periphery (2c) acting as a ring gear, a drive (7) and a plurality of squeeze rollers (3), which can be driven by the drive (7) by means of a gearing (6) with a sun gear (30) anda first planetary gear (16) that is non-rotatably connected to the respective squeeze roller (3), wherein the rotating squeeze rollers (3), when the pump is running, press a hose placed in the pump, while squeezing the hose, against a counter-bearing (4) and thereby convey the medium onward in the conveying direction in the hose, at least one second planetary gear (31), which is configured as a tooth-free friction wheel, being associated with each squeeze roller (3) in addition to the first planetary gear (16), **characterised in that**
- the squeeze rollers (3) are rotatably mounted on a carrier (8) that can be rotated relative to the housing,
- and the second planetary gear (31) is in each case coupled to the inner periphery (2c) of the housing (2) acting as a ring gear in order to make the carrier (8) rotate by means of the drive (7) when the pump is running,
- wherein the second planetary gear (31) of each squeeze roller (3) has, on its outer periphery, a ring (32) made of an elastomeric material and has frictional contact by means of this ring (32) with the smooth inner periphery (2c) of the housing (2).

2. Hose pump according to claim 1, **characterised in that** the sun gear (30) is non-rotatably connected to the drive shaft (10) of the drive (7).

3. Hose pump according to claim 2, **characterised in that** the torque transmitted from the drive shaft (10) to the sun gear (30) is transmitted from the sun gear (30) and the second planetary gear (31) via the inner periphery (2c) of the fixed housing (2) acting as a ring gear to the carrier (8), whereby the carrier (8) is made to rotate when the pump is running even without an inserted hose.

4. Hose pump according to any one of the preceding claims, **characterised in that** the carrier (8) is configured as a carrier disc (8), which is rotatably mounted on a drive shaft (10) of the drive (7) by means of a bearing (33).

5. Hose pump according to claim 4, **characterised in that** the squeeze rollers (3) are in each case arranged on a squeeze roller shaft (9) rotatably mounted in the carrier disc (8), the axis (9') of each squeeze roller shaft (9) running parallel to the drive shaft (10) of the drive (7).

6. Hose pump according to any one of the preceding claims, **characterised in that** the first planetary gear (16) of each squeeze roller (3) is in each case in contact with the sun gear (30) by means of a toothing.

7. Hose pump according to any one of the preceding claims, **characterised in that** the second planetary gear (31) of each squeeze roller (3) is in contact with the inner periphery (2c) of the housing (2) and rolls on the inner periphery (2c) when the pump is running.

8. Hose pump according to any one of claims 4 to 7, **characterised in that** a guide roller (11) is in each case arranged on the carrier disc (8) between two adjacent squeeze rollers (3).

9. Hose pump according to any one of claims 5 to 8, **characterised in that** each of the planetary gears (16, 31) is non-rotatably arranged on the squeeze roller shaft (9) of the respectively associated squeeze roller (3).

10. Hose pump according to any one of the preceding claims, **characterised in that** the hose pump has a threading device for introducing the hose between the squeeze elements (3) and the counter-bearing (4).

11. Hose pump according to claim 10, **characterised in that** the threading device comprises a screw spindle (26), which is rotatably drivable by a spindle drive (28).

12. Hose pump according to claim 11, **characterised in that** the spindle drive (28) is coupled to the drive (7) in such a way that the spindle drive (28) makes the screw spindle (26) rotate as soon as the drive (7) makes the carrier disc (8) rotate.

13. Hose pump according to any one of claims 10 to 12, **characterised in that** the threading device automatically introduces a hose placed therein between the squeeze elements (3) and the counter-bearing (4) when the pump is running.

14. Hose pump according to any one of the preceding claims, **characterised in that** a fixing device is provided to fix a hose to be introduced into the hose pump, wherein the fixing device allows a fixing of the hose at a first point (39) on the inlet side and a second point (40) of the hose pump on the outlet side.

## Revendications

1. Pompe péristaltique (1) servant à refouler un milieu acheminé dans un tuyau flexible, comprenant un boîtier (2), qui présente une périphérie intérieure (2c) agissant en tant que roue à denture intérieure, un système d'entraînement (7) et plusieurs galets d'écrasement (3), qui peuvent être entraînés par le système d'entraînement (7) par l'intermédiaire d'un engrenage (6) pourvu d'un pignon solaire (30) et par un premier pignon satellite (16) relié de manière solidaire en rotation à chaque galet d'écrasement (3), sachant que les galets d'écrasement (3) en rotation poussent un tuyau flexible placé dans la pompe en écrasant ledit tuyau flexible contre un palier complémentaire (4) lorsque la pompe fonctionne et refoulent ce faisant le milieu se trouvant dans le tuyau flexible dans la direction de refoulement, sachant qu'au moins un deuxième pignon satellite (31) est associé à chaque galet d'écrasement (3) outre le premier pignon satellite (16), ledit deuxième pignon satellite étant réalisé sous la forme d'une roue de friction sans denture, **caractérisée en ce que**
- les galets d'écrasement (3) sont installés de manière à pouvoir tourner sur un support (8) pouvant tourner par rapport au boîtier,
- et **en ce que** le deuxième pignon satellite (31) est couplé respectivement à la périphérie intérieure (2c), agissant sous la forme d'une roue à denture intérieure, du boîtier (2) pour la mise en rotation par le système d'entraînement (7) du support (8) lorsque la pompe fonctionne,
- sachant que le deuxième pignon satellite (31) de chaque galet d'écrasement (3) présente, au niveau de sa périphérie extérieure, une bague (32) fabriquée en un matériau élastomère, et se trouve en contact de friction, par l'intermédiaire de ladite bague (32), avec la périphérie intérieure (2c) du boîtier (2), réalisée de manière lisse.

2. Pompe péristaltique selon la revendication 1, **caractérisée en ce que** le pignon solaire (30) est relié de manière solidaire en rotation, à un arbre d'entraînement (10) du système d'entraînement (7).

3. Pompe péristaltique selon la revendication 2, **caractérisée en ce que** le couple de rotation transmis par l'arbre d'entraînement (10) au pignon solaire (30) est transmis par le pignon solaire (30) et par le deuxième pignon satellite (31), par l'intermédiaire de la périphérie intérieure (2c), agissant en tant que roue à denture intérieure, du boîtier (2) immobile, sur le support (8), ce qui permet d'amener en rotation le support (8) même sans tuyau flexible mis en place lorsque la pompe fonctionne.

4. Pompe péristaltique selon l'une quelconque des revendications précédentes, **caractérisée en ce que** le support (8) est réalisé sous la forme d'un disque de support (8), qui est installé au niveau d'un arbre d'entraînement (10) du système d'entraînement (7) de manière à pouvoir tourner sur un palier (33).

5. Pompe péristaltique selon la revendication 4, **caractérisée en ce que** les galets d'écrasement (3) sont disposés respectivement sur un arbre de galet d'écrasement (9) installé de manière à pouvoir tourner dans le disque de support (8), sachant que l'axe (9') de chaque arbre de galet d'écrasement (9) s'étend de manière parallèle par rapport à l'arbre d'entraînement (10) du système d'entraînement (7).

6. Pompe péristaltique selon l'une quelconque des revendications précédentes, **caractérisée en ce que** respectivement le premier pignon satellite (16) de chaque galet d'écrasement (3) se trouve en contact avec le pignon solaire (30) par l'intermédiaire d'une denture.

7. Pompe péristaltique selon l'une quelconque des revendications précédentes, **caractérisée en ce que** le deuxième pignon satellite (31) de chaque galet d'écrasement (3) se trouve en contact avec la périphérie intérieure (2c) du boîtier (2) et roule au niveau de la périphérie intérieure (2c) lorsque la pompe fonctionne.

8. Pompe péristaltique selon l'une quelconque des revendications 4 à 7, **caractérisée en ce que** respectivement un galet de guidage (11) est disposé sur le disque de support (8) entre deux galets d'écrasement (3) adjacents.

9. Pompe péristaltique selon l'une quelconque des revendications 5 à 8, **caractérisée en ce que** chacun des pignons satellites (16, 31) est disposé de manière solidaire en rotation au niveau de l'arbre de galet d'écrasement (9) du galet d'écrasement (3) respectivement associé.

10. Pompe péristaltique selon l'une quelconque des revendications précédentes, **caractérisée en ce que** la pompe péristaltique dispose d'un système d'enfilement servant à introduire la pompe péristaltique entre les éléments d'écrasement (3) et le palier complémentaire (4).

11. Pompe péristaltique selon la revendication 10, **caractérisée en ce que** le système d'enfilement comprend une broche à vis sans fin (26), qui peut être entraînée en rotation par un système d'entraînement de broche (28).

12. Pompe péristaltique selon la revendication 11, **caractérisé en ce que** le système d'entraînement de broche (28) est couplé au système d'entraînement (7) de telle manière que le système d'entraînement de broche (28) amène en rotation la broche à vis sans fin (26) dès que le système d'entraînement (7) amène en rotation le disque de support (8).

13. Pompe péristaltique selon l'une quelconque des revendications 10 à 12, **caractérisée en ce que** le système d'enfilement introduit un tuyau flexible placé dans ce dernier, lorsque la pompe péristaltique fonctionne, de manière autonome entre les éléments d'écrasement (3) et le palier complémentaire (4).

14. Pompe péristaltique selon l'une quelconque des revendications précédentes, **caractérisée en ce qu'**est prévu un système de fixation servant à fixer un tuyau flexible à introduire dans la pompe péristaltique, sachant que le système de fixation permet une fixation du tuyau flexible au niveau d'un premier endroit (39) côté entrée, et au niveau d'un deuxième endroit (40) côté sortie, de la pompe péristaltique.
